# EUROPEAN PATENT APPLICATION

(11) **EP 4 703 378 A1**
(43) Date of publication of application: **04.03.2026**
(21) Application number: 23935119.0
(22) Date of filing: 08.12.2023
(51) Int. Cl.: C07K 16/22, C12N 15/63, C12N 15/11, A61K 39/395, A61P 27/02

(54) **DRUG FOR RELIEVING OCULAR ANGIOGENESIS RELATED DISEASES AND USE THEREOF**

(30) Priority: 25.04.2023 CN 202310456681
(71) Applicant: Nanjing Bionce Biotechnology Co., Ltd., Nanjing, Jiangsu 211500 (CN)
(72) Inventor: LUO, Guangzuo, Nanjing, Jiangsu 211500 (CN); HAN, Xuefei, Nanjing, Jiangsu 211500 (CN); WANG, Yufeng, Nanjing, Jiangsu 211500 (CN); SUN, Dong, Nanjing, Jiangsu 211500 (CN)
(74) Representative: ABG Intellectual Property Law, S.L.
(86) International application number: PCT/CN2023/137432
(87) International publication number: WO 2024/221953

(57) **Abstract**

The present invention belongs to the technical field of biological medicines, and provides a drug for relieving ocular angiogenesis related diseases and the use thereof. A single-chain antibody provided by the present invention has a small molecular weight and relatively good physiological activity, and can more effectively inhibit proliferation of human umbilical vein endothelial cells (HUVECs), thus improving the effect of inhibiting angiogenesis related diseases. In addition, the drug prepared by using a recombinant adeno-associated virus has improved quantity of expression, thus further reducing the treatment cost.

## Description

This application claims the priority of Chinese Patent Application No. 202310456681.1, filed with the China National Intellectual Property Administration on April 25, 2023, and titled with "DRUG FOR RELIEVING OCULAR ANGIOGENESIS RELATED DISEASES AND USE THEREOF", which is hereby incorporated by reference in its entirety.

### FIELD

The present disclosure relates to the technical field of biological medicines, in particular to a drug for relieving ocular neovascularization-related diseases and use thereof.

### BACKGROUND

Ocular neovascularization is a common complication and pathological sequela of multiple ocular diseases and is a major cause of vision loss. According to the anatomical location of abnormal neovascularization, ocular neovascularization can be classified into corneal neovascularization, retinal neovascularization, and choroidal neovascularization. These newly formed blood vessels are leaky, leading to an impairment of retinal function and subsequent accumulation of extracellular fluid. Furthermore, the abnormal neovascularization can interfere with corneal transparency and normal tissue structure. Neovascularization is associated with various ocular diseases, including neovascular age-related macular degeneration (nAMD), diabetic retinopathy (DR), retinopathy of prematurity (ROP), corneal neovascularization, retinal vascular occlusion, and neovascular glaucoma. Among these diseases associated with neovascularization, nAMD and DR are the primary causes of visual impairment.

AMD can be classified as dry AMD or wet AMD, the latter of which is also known as neovascular AMD (nAMD). nAMD is an advanced stage of AMD characterized by the pathological growth of choroidal blood vessels beneath the macula, a condition also known as choroidal neovascularization (CNV). These choroidal blood vessels lack normal vascular structure and function, leading to fluid leakage and ultimately hemorrhagic or leaky retinal detachment. The neovascularization is often accompanied by fibrosis, which causes further damage to retinal cells, particularly photoreceptors. Despite the availability of multiple treatments, nearly half of the patients develop retinal pigment epithelium (RPE) atrophy or macular scar within 2 years, which are major causes of permanent vision loss.

Diabetic retinopathy (DR) is one of the most common microvascular complications of diabetes. Diabetes-related hyperglycemia damages blood vessels of retinal vasculature, including endothelial cells, the basement membrane, and supporting pericytes. Non-proliferative DR (NPDR) is the initial stage of DR, characterized by mild changes such as microaneurysms, microhemorrhages, hard exudates, and cotton wool spots, which can be clinically detected by funduscopy or fundus imaging. When lesions involve the macula, it can manifest as diffuse or focal vascular leakage in the macular region (such as hard exudates and dot hemorrhages); progressive vascular lesions, including microangiomas, intraretinal hemorrhages, vascular tortuosity, and vascular malformations, ultimately leading to abnormal capillary formation; and retinal capillary occlusion which can lead to varying degrees of vision loss.

Retinopathy of prematurity (ROP) is a condition in premature infants, born at less than 36 weeks of gestation, with low birth weight, and exposed to prolonged oxygen therapy, in whom the unvascularized retina develops fibrovascular proliferation and contraction, which further causes tractional retinal detachment and blindness. It is primarily caused by vasoconstriction and vasoproliferation of the incompletely vascularized retina in response to oxygen, and clinically, it includes an active phase and a fiber membrane formation phase.

The common vasoactive molecule that drives neovascularization in the corneal, retinal, and choroidal vascular beds is VEGF, which is considered the most critical regulator of ocular neovascularization; it regulates cell proliferation, survival, motion, and vascular permeability. Multiple clinical studies have shown that abnormally high levels of VEGF are found in several ocular diseases, including nAMD, DR, diabetic macular edema (DME), ROP, and neovascular glaucoma. Intravitreal injections of anti-VEGF drugs, including bevacizumab, ranibizumab, aflibercept, conbercept, and brolucizumab, are widely used to inhibit ocular neovascularization, and anti-VEGF therapy is currently the first-line treatment strategy for nAMD, DR, and ROP. Due to the potential to reduce the treatment frequency and lessen the drug burden, gene therapy drugs have become a key direction for ophthalmic therapeutic drugs in recent years. rAAV vectors have become the most widely used viruses in international clinical trials for gene therapy due to their characteristics such as diverse serotypes, extremely low immunogenicity, high safety, broad host cell range, strong diffusion ability, and long-term gene expression in vivo. Currently, rAAV vectors have been used to deliver genes encoding monoclonal antibodies (mAbs) to achieve long-term expression in vivo, thereby reducing the number of administrations required for traditional monoclonal antibody injections. However, the in vivo expression levels of gene therapy drugs used in the prior art remain very limited, and improvements on it are currently focused mainly on the modification of adeno-associated viruses and the optimization of the amino acid sequence of VEGF antibodies. There is still an urgent clinical need for gene therapy drugs for diseases related to ocular neovascularization that allow for high-level expression and are more effective.

### SUMMARY

In view of the foregoing, the technical problem to be solved by the present disclosure is to provide use of a single-chain antibody and a nucleic acid encoding the single-chain antibody in the manufacture of a medicament for ameliorating ocular neovascularization-related diseases.

The anti-VEGF single-chain antibody provided in the present disclosure comprises:
a light chain comprising three CDRs, and at least one of the CDRs has an amino acid sequence as set forth in SEQ ID NO: 1, 2 or 3, or an amino acid sequence sharing a sequence identity of at least 80% (preferably 85%, 90%, 95%, 98% or 99%) with SEQ ID NO: 1, 2 or 3; and
a heavy chain comprising three CDRs, and at least one of the CDRs has an amino acid sequence as set forth in SEQ ID NO: 4, 5 or 6, or an amino acid sequence sharing a sequence identity of at least 80% (preferably 85%, 90%, 95%, 98% or 99%) with SEQ ID NO: 4, 5 or 6.

In some embodiments, the anti-VEGF single-chain antibody comprises three light chain CDRs having amino acid sequences as set forth in SEQ ID NOs: 1, 2 and 3, respectively; and three heavy chain CDRs having amino acid sequences as set forth in SEQ ID NOs: 4, 5 and 6, respectively.

In some specific embodiments, the anti-VEGF single-chain antibody comprises a light chain variable region, a linker, and a heavy chain variable region; wherein the heavy chain variable region has an amino acid sequence as set forth in SEQ ID NO: 7; and the light chain variable region has an amino acid sequence as set forth in SEQ ID NO: 8.

In a specific embodiment, the anti-VEGF single-chain antibody comprises, from N-terminal to C-terminal, a secretory signal peptide, a light chain variable region, a linker, and a heavy chain variable region. The incorporation of the secretory signal peptide provided in the present disclosure elevates gene expression level, more effectively inhibits proliferation of human umbilical vein endothelial cells (HUVEC), enables long-term and effective inhibition of neovascularization through the expression of anti-neovascularization protein antiVEGFscFv delivered via an adeno-associated virus (AAV) vector, thereby reducing administration frequency, improving patient compliance to the therapy, and reducing risk and trauma of the treatment. In the present disclosure, the secretory signal peptide has an amino acid sequence as set forth in SEQ ID NO: 9. Experiment results show that compared to other secretory signal peptides, the signal peptide of the present disclosure is more advantageous for increasing the expression level of the anti-VEGF single-chain antibody. In the present disclosure, the linker is used to link the two variable regions of the single-chain antibody, and the linker may be (G₄S)ₙ, wherein n is 2 to 10; in some embodiments, the linker is (G₄S)₄.

Furthermore, the present disclosure further provides a nucleic acid encoding the aforementioned anti-VEGF single-chain antibody.

The nucleic acid provided in the present disclosure comprises a nucleotide sequence encoding the light chain variable region, and a nucleotide sequence encoding the heavy chain variable region. The nucleic acid may be DNA, RNA, cDNA or PNA. The nucleic acid may be single-stranded or double-stranded. The nucleic acid may comprise nucleotide sequences with different functions, such as a coding region, a non-coding region such as a regulatory sequence (for example a promoter or a transcription terminator). The nucleic acid may be topologically linear or circular. The nucleic acid can be part of a vector (for example an expression or cloning vector), or a combination of one or more fragments. The nucleic acid may be obtained from natural sources, or prepared using recombinant, enzymatic or chemical technologies. The RNA may be an mRNA obtained by transcription of a gene, and the like. In some embodiments, the nucleic acid is a codon-optimized DNA. In some embodiments, the nucleotide sequence encoding the light chain variable region is as set forth in SEQ ID NO: 10; and the nucleotide sequence encoding the heavy chain variable region is as set forth in SEQ ID NO: 11. More specifically, the nucleic acid encoding the anti-VEGF single-chain antibody (including the signal peptide and the antibody amino acid sequence) provided in the present disclosure has a nucleotide sequence as set forth in SEQ ID NO: 12. Compared to nucleic acids without optimization or with other optimization, the nucleic acid provided in the present disclosure can achieve a better ocular expression, and thereby exhibits more effective therapeutic effects.

Furthermore, the present disclosure further provides an expression unit comprising a promoter and the aforementioned nucleic acid. The expression unit may further comprise a terminator. A regulatory segment may be included on either side of or between the promoter and the terminator. The regulatory segment may comprise a promoter, an enhancer, a transcription termination signal, a polyadenylation sequence, a replication origin, a restriction site, and a homologous recombination site, which are operably linked to the nucleic acid, such as an enhancer of a promoter, and a poly(A) signal. In some embodiments of the present disclosure, the promoter is a chicken β-actin (CBA) promoter. Compared to other promoters, CBA is more favorable for increasing the expression level of the anti-VEGF single-chain antibody, thereby improving the effect of inhibiting the proliferation of human umbilical vein endothelial cells (HUVEC), and more effectively inhibiting neovascularization.

The present disclosure further provides a recombinant vector comprising the aforementioned nucleic acid or expression unit.

In some embodiments, the recombinant vector successively comprises a 5' terminal inverted repeat sequence, a promoter sequence, the aforementioned nucleic acid, a T2A sequence, a BGHpolyA signal sequence, and a 3' terminal inverted repeat sequence. The vector of the present disclosure has a backbone derived from sources such as plants, animals, bacteria, fungi, phages or viruses, which are not limited in the present disclosure. The viral vector includes: an adenovirus vector, an adeno-associated virus vector, a retrovirus vector, or a lentivirus vector, and the like. The present disclosure uses an adeno-associated virus (AAV) vector as a backbone for expression of single-chain antibody. In some specific embodiments, the backbone vector of the recombinant vector is pAAV2.

The present disclosure further provides a combination of plasmids, comprising the aforementioned recombinant vector and a helper plasmid for virus packaging. In the present disclosure, the helper plasmid for virus packaging includes a Helper plasmid and a Rep-Cap2 plasmid.

The present disclosure further provides a host comprising the aforementioned nucleic acid integrated into its genome, or a host that is transfected or transformed with the aforementioned recombinant vector or the plasmid combination.

The host provided by the present disclosure is used to preserve or amplify the aforementioned nucleic acid, or to construct and express the aforementioned adeno-associated virus, which are not limited in the present disclosure. In the present disclosure, the host is a prokaryote or an animal cell. The prokaryote includes but is not limited to *E.coli,* and the animal cell is at least one selected from the group consisting of CHO, BHK, Sp2/0, HEK-293, HEK293T, Hep G2, HELA, CHO-K1, COS-1, COS-7, NIH3T3, A204, A549, D-407, CHO, HCS-2, HT-29, U87, Sf9, and FD-CHOS cells. In some specific embodiments, HEK293T cells are used as a host for virus packaging.

The present disclosure further provides a recombinant adeno-associated virus obtained by culturing the host provided by the present disclosure.

The present disclosure further provides a method for constructing a recombinant adeno-associated virus, comprising constructing an AAV2-antiVEGFscFv recombinant plasmid; detecting anti-VEGF expression by transfecting cells with the plasmid in vitro; packaging an AAV2-antiVEGFscFv virus; and harvesting the recombinant adeno-associated virus via transfecting cells with the virus in vitro.

More specifically, the construction method comprises:
S1. constructing a recombinant plasmid pAAV2-antiVEGFscFv: (1) double-digesting an antiVEGFscFv plasmid with BglII and BsrGI restriction endonucleases, and after digesting at 37°C overnight, recovering an insert fragment using a gel recovery and purification kit; (2) double-digesting a vector, AAV2-GFP, with BglII and BsrGI restriction endonucleases, and after digesting at 37°C overnight, recovering a vector using a gel recovery and purification kit; (3) ligating the vector and the insert fragment at a molar ratio of 1:3 using T4 DNA ligase enzyme , transforming, plating, and culturing overnight at 37°C; and (4) picking single colonies for overnight shaking culture, extracting plasmids using a plasmid miniprep kit, identifying the plasmids by restriction digestion, and sending successfully identified plasmids to a company for sequencing, wherein a successful construction of the plasmid is confirmed by successful sequence alignment;
S2. co-transfecting cells with a pAAV2-H-antiVEGFscFv (containing an SPH secretory signal peptide) plasmid and a pAAV2-antiVEGFscFv (containing an SPL secretory signal peptide) plasmid: 293T cells are passaged into a 10 cm dish and transfection is performed after cell growth is stabilized; the transfection method is as follows: 1.5 mL EP tubes are labeled; Tube A: 10 µg of an AAV2-antiVEGFscFv plasmid and 480 µL of Opti-MEM are added, mixed, and allowed to stand at room temperature for 5 min; Tube B: 20 µg of PEI and 480 µL of Opti-MEM are added and mixed; the liquid in Tube A is added to Tube B, mixed, and allowed to stand at room temperature for 15 min; then the liquid is gently mixed and added to a 10 cm dish, which is then placed in a 37°C incubator for 2 to 3 days, and the cells are harvested for ELISA and Western blot analysis;
S3. expressing an antiVEGF protein in vitro via pAAV2-H-antiVEGFscFv and pAAV2-antiVEGFscFv; and
S4. packaging and purifying the virus.

The method further comprises steps of detecting expression of an antiVEGF protein by the AAV2-antiVEGFscFv virus, and detecting an inhibitory effect of the AAV2-antiVEGFscFv virus on HUVEC cell functions; and steps of detecting, through in vivo experiments, the expression of the AAV2-antiVEGFscFv virus in the eyes of animal models and the inhibitory effect of the AAV2-antiVEGFscFv virus on ocular neovascularization in the animal models.

Furthermore, the present disclosure further provides use of any one of I) to VII), in the manufacture of a medicament for ameliorating ocular neovascularization-related diseases:
I) the aforementioned anti-VEGF single-chain antibody;
II) the aforementioned nucleic acid;
III) the aforementioned expression unit;
IV) the aforementioned recombinant vector;
V) the aforementioned plasmid combination
VI) the aforementioned host; and
VII) the aforementioned recombinant adeno-associated virus.

In the present disclosure, the ocular neovascularization-related diseases include neovascular age-related macular degeneration, diabetic retinopathy, retinopathy of prematurity, corneal neovascularization, retinal vascular occlusion, and/or neovascular glaucoma.

The present disclosure further provides a medicament for ameliorating ocular neovascularization-related diseases, comprising the recombinant adeno-associated virus provided in the present disclosure.

The medicament of the present disclosure is a novel gene therapy drug based on an AAV vector for inhibiting ocular neovascularization. The medicament comprises an AAV2 vector carrying an antiVEGFscFv gene expression cassette, wherein a novel secretory signal peptide sequence is inserted to further enhance the gene expression level thereof, and the medicament can also inhibit proliferation of human umbilical vein endothelial cells (HUVECs).

The medicament of the present disclosure comprises the aforementioned recombinant adeno-associated virus, and further comprises a pharmaceutically acceptable excipient. In some embodiments, the recombinant adeno-associated virus has a titer of 1×10⁹ GC to 2×10⁹ GC. The pharmaceutically acceptable excipient includes, but is not limited to, a buffer, an osmotic regulator, an antimicrobial agent, or a preservative. The medicament of the present disclosure further comprises additional drug for treating a disease related to ocular neovascularization. In some embodiments, the medicament of the present disclosure is in a dosage form of an injection, which includes a solution for injection or a powder for injection, to which the present disclosure is not limited. The injection is an intravitreal injection, a subretinal injection, or a suprachoroidal injection.

The present disclosure further provides a method for ameliorating a disease related to ocular neovascularization, comprising administering the medicament of the present disclosure. In the present disclosure, the method comprises injection, wherein the injection includes intravitreal injection, subretinal injection, or suprachoroidal injection. The injection is performed at an amount from 2 µL/eye to 4 µL/eye.

The present disclosure provides an anti-VEGF single-chain antibody, an expression unit, a recombinant vector, a host, and a recombinant adeno-associated virus vector, for inhibiting ocular neovascularization, and further provides a production method, use, and a medicament thereof. Compared to the prior art, the present disclosure has at least one of the following advantages:
1. The single-chain antibody provided by the present disclosure has a small molecular weight and good physiological activity, can more effectively inhibit the proliferation of human umbilical vein endothelial cells (HUVEC), and improves the effect in inhibiting diseases related to neovascularization.
2. The nucleic acid provided by the present disclosure is codon-optimized, which increases the expression level of target gene, and a novel gene therapy medicament for inhibiting ocular neovascularization based on an AAV vector is provided; the medicament comprises an AAV2 vector carrying an antiVEGFscFv gene expression cassette, and a novel secretion signal peptide sequence is inserted to further increase the gene expression level, and the proliferation of human umbilical vein endothelial cells (HUVEC) is also inhibited; the expression of the anti-neovascularization protein antiVEGFscFv delivered via an adeno-associated virus (AAV) vector can provide long-term and effective inhibition on neovascularization, reducing the frequency of administration, while also effectively improving patient compliance with treatment, and reducing treatment risks and trauma.
3. The present disclosure involves: constructing anAAV2-antiVEGFscfv recombinant plasmid; detecting anti-VEGF expression through in vitro cell plasmid transfection; packaging an AAV2-antiVEGFscfv virus; detecting the expression of the antiVEGF protein by the AAV2-antiVEGF virus through in vitro cell virus transduction, and detecting the inhibitory effect of the AAV2-antiVEGFscfv virus on HUVEC cell function; and detecting the expression of the AAV2-antiVEGFscfv virus in the eyes of animal models and the inhibitory effect of the AAV2-antiVEGFscfv virus on ocular neovascularization in animal models through in vivo experiments.
4. The recombinant adeno-associated virus used in the present disclosure can significantly inhibit the growth of ocular neovascularization. The expression level of antiVEGF is more than 20 times higher in cells infected with AAV2-antiVEGFscfv carrying an SPL secretion signal peptide than that with AAV2-H-antiVEGFscfv carrying an SPH secretion signal peptide. This significantly reduces treatment costs, avoids side effects caused by repeated injections of antibody drugs, and has high safety, providing a new clinical treatment method and strategy for inhibiting diseases related to ocular neovascularization.

### BRIEF DESCRIPTION OF DRAWINGS

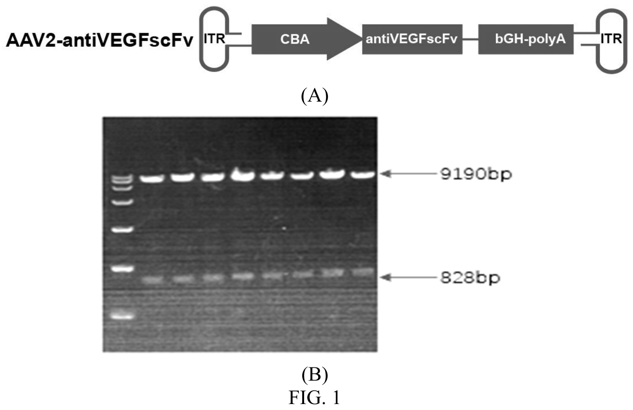

FIG. 1 shows the construction of a recombinant adeno-associated virus AAV2-antiVEGFscFv plasmid in an example of the present disclosure; wherein, FIG. 1A is a map of the AAV2-antiVEGFscFv plasmid; and FIG. 1B is an electrophoresis gel image for restriction digestion analysis of the plasmid;

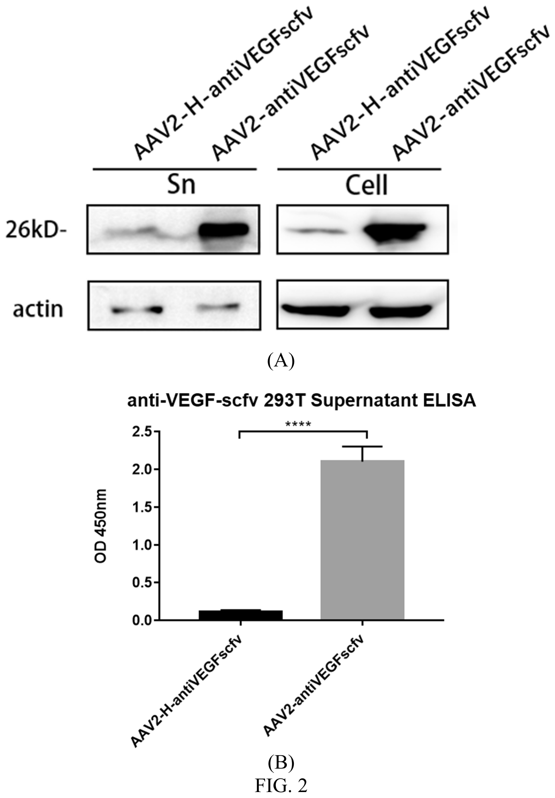

FIG. 2 shows the detection of the expression of the antiVEGFscFv protein from the AAV2-antiVEGFscFv plasmid by plasmid transfection in an example of the present disclosure; wherein, FIG. 2A shows the detection by Western Blot of the expression of antiVEGFscFv in cells and in the culture supernatant after 293T cells were transfected with the AAV2-antiVEGFscFv plasmid; and FIG. 2B shows the verification by ELISA of the expression of antiVEGFscFv in cells and in the culture supernatant after 293T cells were transfected with an AAV2-anti-VEGF (without a secretion signal peptide) plasmid and an AAV2-antiVEGFscFv plasmid, respectively;

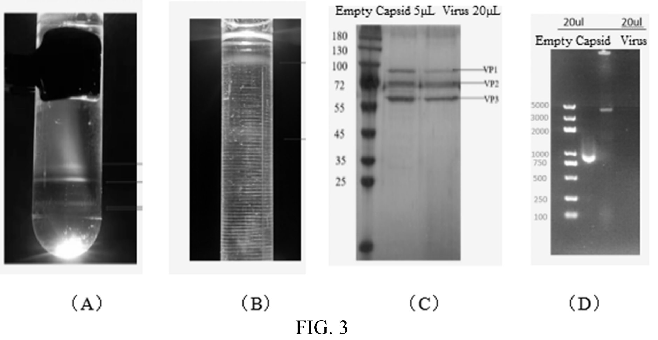

FIG. 3 shows the results of cesium chloride density gradient centrifugation of an adeno-associated virus and the detection of virus purity and titer in an example of the present disclosure; wherein, FIG. 3A shows the first cesium chloride density gradient centrifugation; FIG. 3B shows the second cesium chloride density gradient centrifugation; FIG. 3C shows detection of the virus capsid by silver staining; and FIG. 3D shows detection of the virus genome by agarose gel electrophoresis;

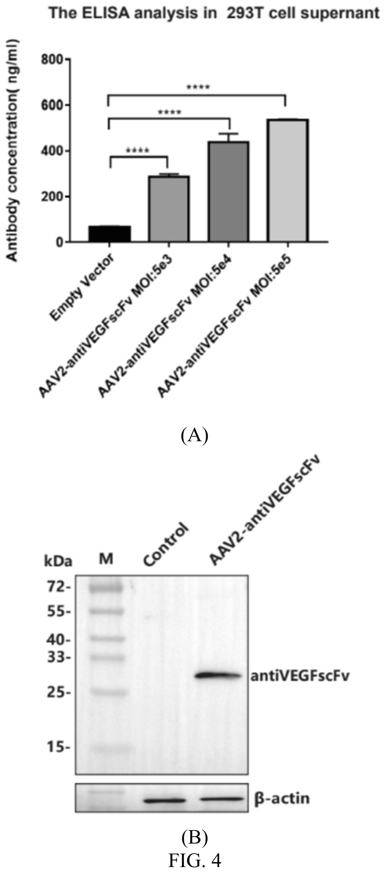

FIG. 4 shows the detection of the expression of the antiVEGFscFv protein in cell culture supernatant of 293T cells infected with packaged AAV2-H-antiVEGFscFv and AAV2-antiVEGFscFv viruses in an example of the present disclosure; wherein, FIG. 4A shows detection by ELISA of the expression of secreted antiVEGFscFv in the supernatant (****, P<0.001); and FIG. 4B shows the detection by Western Blot of the expression of secreted antiVEGFscFv in the supernatant;

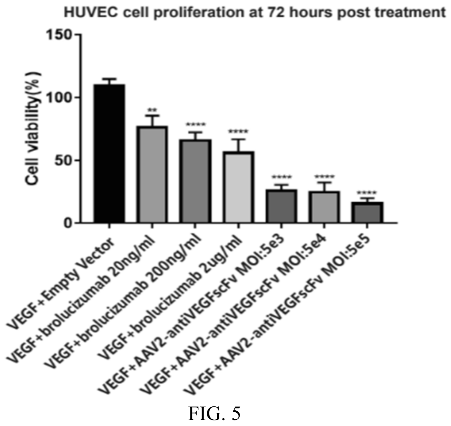

FIG. 5 shows a cell proliferation experiment in an example of the present disclosure, in which the inhibitory effect of secreted antiVEGFscFv in the supernatant on cell proliferation is confirmed by adding VEGF, Beo antibody, AAV2-antiVEGFscFv, and an empty vector to the supernatant of HUVEC cells;

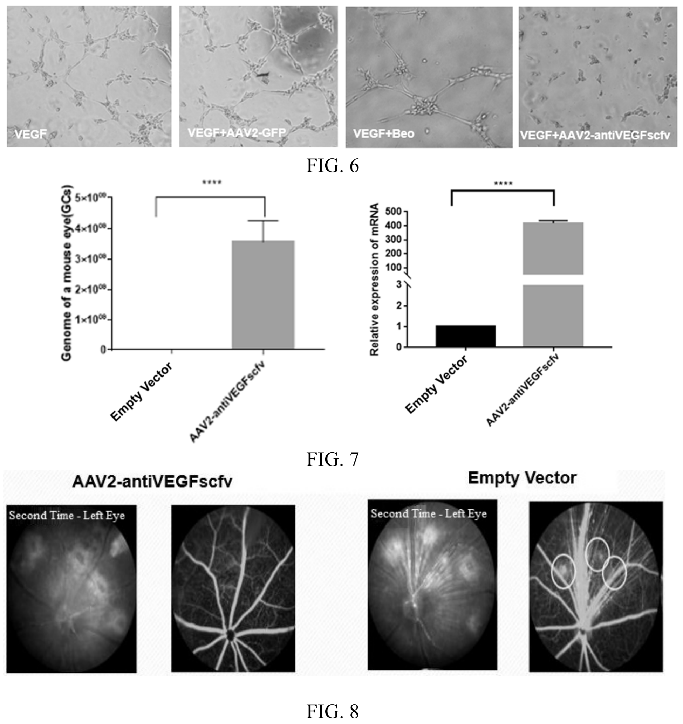

FIG. 6 shows a tube formation experiment in an example of the present disclosure, in which the inhibitory effect of secreted antiVEGFscFv in the 293T cell supernatant on tube formation is confirmed by adding VEGF, Beo antibody, AAV2-antiVEGFscFv, and an empty vector to the supernatant of HUVEC cells grown on matrigel;
FIG. 7 shows an in vivo detection experiment of the genome and RNA in mice in an example of the present disclosure, in which 2 µL (5e8) of AAV2-antiVEGFscFv/empty vector virus was first injected into the vitreous cavity of mice, and two weeks later the mouse eyeballs were taken out; wherein, FIG. 7A shows absolute quantification performed after extracting the mouse eyeball genome (****, P<0.001); and FIG. 7B shows RT-PCR performed after extracting the mouse eyeball RNA (****, P<0.001);
FIG. 8 shows fundus photography and fluorescein angiography in laser-induced choroidal neovascularization (CNV) model mice two weeks after intravitreal injection of 2 µL (5e8) of AAV2-antiVEGFscFv/empty vector virus in an example of the present disclosure, demonstrating the inhibitory effect of AAV2-antiVEGFscFv on neovascularization in the mouse laser-induced model;

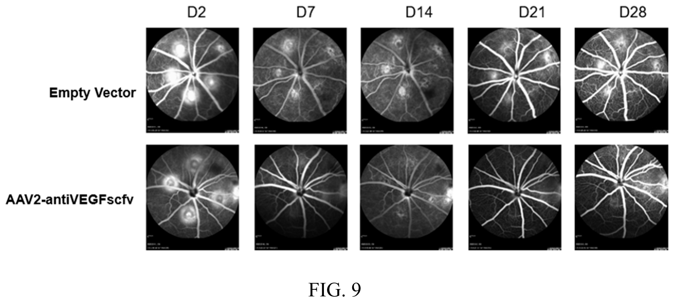

FIG. 9 shows that in an example of the present disclosure, a laser-induced choroidal neovascularization (CNV) model rat was unilaterally injected with 4 µL (1e9) of AAV2-antiVEGFscFv virus or an empty vector in the vitreous cavity of one eye, and was injected with an equal volume of PBS in the contralateral eye; two weeks later, lesions were induced by laser to cause neovascularization in the rat eyes; and fluorescein angiography was performed on D2, D7, D14, D21, and D28 after laser induction to demonstrate the inhibitory effect of AAV2-antiVEGFscFv on neovascularization in the rat laser-induced model;

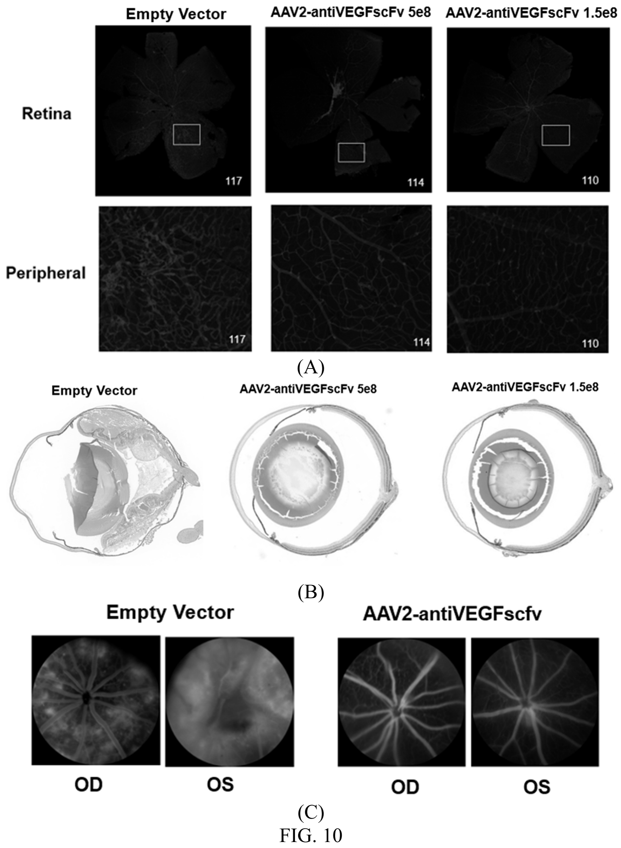

FIG. 10 shows the inhibitory effect of AAV2-antiVEGFscFv on neovascularization in Rho-rtTA/Tet-VEGFA double transgenic model mice in an example of the present disclosure, in which eyeballs were taken out two weeks after the intravitreal injection of 2 µL (5e8) of AAV2-antiVEGFscFv or empty vector virus; wherein, FIG. 10A shows the inhibitory effect on neovascularization by retinal flat-mount and lectin staining; FIG. 10B shows the inhibitory effect on retinal detachment by eye tissue sections and immunofluorescence; and FIG. 10C shows the inhibitory effect of the virus on vascular leakage by fluorescein angiography;

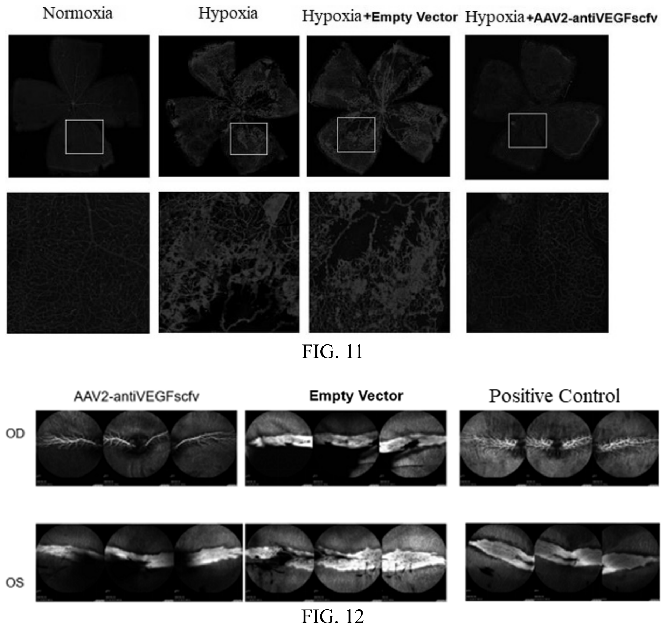

FIG. 11 shows the inhibitory effect of AAV2-antiVEGFscFv on neovascularization in an oxygen-induced retinopathy (OIR) mouse model in an example of the present disclosure, in which eyeballs were taken out 5 days after intravitreal injection of 1 µL (2.5e8) of AAV2-antiVEGFscFv virus or an empty vector virus in the vitreous cavity; the figure shows the inhibition of neovascularization as demonstrated by retinal flat-mount and lectin staining; and
FIG. 12 shows the the inhibitory effect of AAV2-antiVEGFscFv on retinal neovascularization and leakage in a VEGF-induced rabbit model of retinal vascular leakage in an example of the present disclosure, in which 50 µL (1.5e10) of AAV2-antiVEGFscFv virus, an empty vector virus, or a positive control (an anti-neovascularization antibody drug) was subretinally injected on D0; VEGF was subretinally injected on D14 to induce retinal vascular leakage; and fluorescein angiography was performed on D21 to demonstrate the inhibitory effect of the virus on neovascularization and leakage.

### DETAILED DESCRIPTION

The present disclosure provides a medicament for ameliorating diseases related to ocular neovascularization and uses thereof, and a person skilled in the art can implement the same by referring to the content herein and appropriately modifying the process parameters. It should be particularly noted that all similar substitutions and modifications are obvious to those skilled in the art and are deemed to be included within the scope of the present disclosure. The method and application of the present disclosure are described by way of preferred embodiments, and it is obvious that those skilled in the art can make modifications or appropriate changes and combinations to the method and application herein without departing from the content, spirit, and scope of the present disclosure, to realize and apply the technology of the present disclosure.

All reagents used in the present disclosure are common commercially available products and can be purchased from the market.

In the present disclosure, the VEGF single-chain antibody comprises CDRs and FR regions having the following sequences:

| **Name** | **Sequence** |
|---|---|
| FR-L1 | EIVMTQSPSTLSASVGDRVIITC |
| CDR-L1 | QASEIIHSWLA (SEQ ID NO: 1) |
| FR-L2 | WYQQKPGKAPKLLIY |
| CDR-L2 | LASTLAS (SEQ ID NO: 2) |
| FR-L3 | GVPSRFSGSGSGAEFTLTISSLQPDDFATYYC |
| CDR-L3 | QNVYLASTNGAN (SEQ ID NO: 3) |
| FR-L4 | FGQGTKLTVL |
| FR-H1 | EVQLVESGGGLVQPGGSLRLSCTASGFSLT |
| CDR-H1 | DYYYMT (SEQ ID NO: 4) |
| FR-H2 | WVRQAPGKGLEWVG |
| CDR-H2 | FIDPDDDPYYATWAKG (SEQ ID NO: 5) |
| FR-H3 | RFTISRDNSKNTLYLQMNSLRAEDTAVYYCAG |
| CDR-H3 | GDHNSGWGLDI (SEQ ID NO: 6) |
| FR-H4 | WGQGTLVTVSS |

The anti-VEGF single-chain antibody comprises a light chain variable region having the sequence of:

EIVMTQSPSTLSASVGDRVIITCQASEIIHSWLAWYQQKPGKAPKLLIYLASTL
ASGVPSRFSGSGSGAEFTLTISSLQPDDFATYYCQNVYLASTNGANFGQGTKLTVL
(SEQ ID NO: 7).

A nucleic acid encoding the light chain variable region of the anti-VEGF single-chain antibody has a nucleotide sequence of:

GAGATCGTGATGACCCAGAGCCCCAGCACCCTGAGCGCCAGCGTGGGCG
ACAGGGTGATCATCACCTGCCAGGCCAGCGAGATCATCCACAGCTGGCTGGCCTG
GTACCAGCAGAAGCCCGGCAAGGCCCCCAAGCTGCTGATCTACCTGGCCAGCACC
CTGGCCAGCGGCGTGCCCAGCAGGTTCAGCGGCAGCGGCAGCGGCGCCGAGTTC
ACCCTGACCATCAGCAGCCTGCAGCCCGACGACTTCGCCACCTACTACTGCCAGA
ACGTGTACCTGGCCAGCACCAACGGCGCCAACTTCGGCCAGGGCACCAAGCTGA
CCGTGCTG (SEQ ID NO: 10).

The anti-VEGF single-chain antibody comprises a heavy chain variable region having the sequence of:

EVQLVESGGGLVQPGGSLRLSCTASGFSLTDYYYMTWVRQAPGKGLEWVG
FIDPDDDPYYATWAKGRFTISRDNSKNTLYLQMNSLRAEDTAVYYCAGGDHNSGWG
LDI WGQGTLVTVSS (SEQ ID NO: 8).

A nucleic acid encoding the light chain variable region of the anti-VEGF single-chain antibody has a nucleotide sequence of:

GAGGTGCAGCTGGTGGAGAGCGGCGGCGGCCTGGTGCAGCCCGGCGGC
AGCCTGAGGCTGAGCTGCACCGCCAGCGGCTTCAGCCTGACCGACTACTACTACA
TGACCTGGGTGAGGCAGGCCCCCGGCAAGGGCCTGGAGTGGGTGGGCTTCATCG
ACCCCGACGACGACCCCTACTACGCCACCTGGGCCAAGGGCAGGTTCACCATCAG
CAGGGACAACAGCAAGAACACCCTGTACCTGCAGATGAACAGCCTGAGGGCCGA
GGACACCGCCGTGTACTACTGCGCCGGCGGCGACCACAACAGCGGCTGGGGCCTG
GACATCTGGGGCCAGGGCACCCTGGTGACCGTGAGCAGCTGA (SEQ ID NO: 11).

The secretory peptide has an amino acid sequence of:
METHSQVFVYMLLCLSGVEG (SEQ ID NO: 9).

A nucleic acid encoding the secretory peptide has a nucleotide sequence of:

ATGGAGACACATTCTCAGGTCTTTGTATACATGTTGCTGTGTTTGTCTGGT
GTCGAGGGC.

The full length anti-VEGF single-chain antibody has an amino acid sequence of:

EIVMTQSPSTLSASVGDRVIITCQASEIIHSWLAWYQQKPGKAPKLLIYLASTL
ASGVPSRFSGSGSGAEFTLTISSLQPDDFATYYCQNVYLASTNGANFGQGTKLTVLGG
GGGSGGGGSGGGGSGGGGSEVQLVESGGGLVQPGGSLRLSCTASGFSLTDYYYMTW
VRQAPGKGLEWVGFIDPDDDPYYATWAKGRFTISRDNSKNTLYLQMNSLRAEDTAVY
YCAGGDHNSGWGLDIWGQGTLVTVSS.

The full length anti-VEGF single-chain antibody with the secretory signal peptide has an amino acid sequence of:

METHSQVFVYMLLCLSGVEGEIVMTQSPSTLSASVGDRVIITCQASEIIHSWL
AWYQQKPGKAPKLLIYLASTLASGVPSRFSGSGSGAEFTLTISSLQPDDFATYYCQNV
YLASTNGANFGQGTKLTVLGGGGGSGGGGSGGGGSGGGGSEVQLVESGGGLVQPGG
SLRLSCTASGFSLTDYYYMTWVRQAPGKGLEWVGFIDPDDDPYYATWAKGRFTISRD
NSKNTLYLQMNSLRAEDTAVYYCAGGDHNSGWGLDIWGQGTLVTVSS.

A nucleic acid encoding the full length anti-VEGF single-chain antibody with the secretory signal peptide has a nucleotide sequence of:

ATGGAGACACATTCTCAGGTCTTTGTATACATGTTGCTGTGTTTGTCTGGT
GTCGAGGGCGAGATCGTGATGACCCAGAGCCCCAGCACCCTGAGCGCCAGCGTG
GGCGACAGGGTGATCATCACCTGCCAGGCCAGCGAGATCATCCACAGCTGGCTGG
CCTGGTACCAGCAGAAGCCCGGCAAGGCCCCCAAGCTGCTGATCTACCTGGCCAG
CACCCTGGCCAGCGGCGTGCCCAGCAGGTTCAGCGGCAGCGGCAGCGGCGCCGA
GTTCACCCTGACCATCAGCAGCCTGCAGCCCGACGACTTCGCCACCTACTACTGCC
AGAACGTGTACCTGGCCAGCACCAACGGCGCCAACTTCGGCCAGGGCACCAAGC
TGACCGTGCTGGGCGGCGGCGGCAGCGGCGGCGGCAGCGGCGGCGGCG
GCAGCGGCGGCGGCGGCAGCGAGGTGCAGCTGGTGGAGAGCGGCGGCGGCCTGG
TGCAGCCCGGCGGCAGCCTGAGGCTGAGCTGCACCGCCAGCGGCTTCAGCCTGAC
CGACTACTACTACATGACCTGGGTGAGGCAGGCCCCCGGCAAGGGCCTGGAGTGG
GTGGGCTTCATCGACCCCGACGACGACCCCTACTACGCCACCTGGGCCAAGGGCA
GGTTCACCATCAGCAGGGACAACAGCAAGAACACCCTGTACCTGCAGATGAACAG

CCTGAGGGCCGAGGACACCGCCGTGTACTACTGCGCCGGCGGCGACCACAACAG
CGGCTGGGGCCTGGACATCTGGGGCCAGGGCACCCTGGTGACCGTGAGCAGCTG
A（SEQ ID NO: 12）.

The promoter is a chicken β-actin promoter having a nucleotide sequence of:

TTATTAATAGTAATCAATTACGGGGTCATTAGTTCATAGCCCATATATGGAG
TTCCGCGTTACATAACTTACGGTAAATGGCCCGCCTGGCTGACCGCCCAACGACCC
CCGCCCATTGACGTCAATAATGACGTATGTTCCCATAGTAACGCCAATAGGGACTTT
CCATTGACGTCAATGGGTGGAGTATTTACGGTAAACTGCCCACTTGGCAGTACATC
AAGTGTATCATATGCCAAGTACGCCCCCTATTGACGTCAATGACGGTAAATGGCCC
GCCTGGCATTATGCCCAGTACATGACCTTATGGGACTTTCCTACTTGGCAGTACATC
TACGTATTAGTCATCGCTATTACCATGGTCGAGGTGAGCCCCACGTTCTGCTTCACT
CTCCCCATCTCCCCCCCCTCCCCACCCCCAATTTTGTATTTATTTATTTTTTAATTATT
TTGTGCAGCGATGGGGGCGGGGGGGGGGGGGGCGCGCGCCAGGCGGGGCGG
GGCGGGGCGAGGGGCGGGGCGGGGCGAGGCGGAGAGGTGCGGCGGCAGCCAAT
CAGAGCGGCGCGCTCCGAAAGTTTCCTTTTATGGCGAGGCGGCGGCGGCGGC
CCTATAAAAAGCGAAGCGCGCGGCGGGCGGGAGTCGCTGCGCGCTGCCTTCGCCC
CGTGCCCCGCTCCGCCGCCGCCTCGCGCCGCCCGCCCCGGCTCTGACTGACCGCG
TTACTCCCACAGGTGAGCGGGCGGGACGGCCCTTCTCCTCCGGGCTGTAATTAGCG
CTTGGTTTAATGACGGCTTGTTTCTTTTCTGTGGCTGCGTGAAAGCCTTGAGGGGC
TCCGGGAGGGCCCTTTGTGCGGGGGGAGCGGCTCGGGGGGTGCGTGCGTGTGTGT
GTGCGTGGGGAGCGCCGCGTGCGGCTCCGCGCTGCCCGGCGGCTGTGAGCGCTGC
GGGCGCGGCGCGGGGCTTTGTGCGCTCCGCAGTGTGCGCGAGGGGAGCGCGGCC
GGGGGCGGTGCCCCGCGGTGCGGGGGGGGCTGCGAGGGGAACAAAGGCTGCGTG
CGGGGTGTGTGCGTGGGGGGGTGAGCAGGGGGTGTGGGCGCGTCGGTCGGGCTG
CAACCCCCCCTGCACCCCCCTCCCCGAGTTGCTGAGCACGGCCCGGCTTCGGGTG
CGGGGCTCCGTACGGGGCGTGGCGCGGGGCTCGCCGTGCCGGGCGGGGGGTGGC
GGCAGGTGGGGGTGCCGGGCGGGGCGGGGCCGCCTCGGGCCGGGGAGGGCTCGG
GGGAGGGGCGCGGCGGCCCCCGGAGCGCCGGCGGCTGTCGAGGCGCGGCGAGCC
GCAGCCATTGCCTTTTATGGTAATCGTGCGAGAGGGCGCAGGGACTTCCTTTGTCC
CAAATCTGTGCGGAGCCGAAATCTGGGAGGCGCCGCCGCACCCCCTCTAGCGGGC
GCGGGGCGAAGCGGTGCGGCGCCGGCAGGAAGGAAATGGGCGGGGAGGGCCTTC
GTGCGTCGCCGCGCCGCCGTCCCCTTCTCCCTCTCCAGCCTCGGGGCTGTCCGCGG

GGGGACGGCTGCCTTCGGGGGGGACGGGGCAGGGCGGGGTTCGGCTTCTGGCGT
GTGACCGGCGGCTCTAGACAATTGTACTAACCTTCTTCTCTTTCCTCTCCTGACAG
GTTGG.

The present disclosure is further illustrated in conjunction with the following examples:

### Example 1

Construction of recombinant plasmid pAAV2-antiVEGFscFv (a schematic map of the plasmid is shown in FIG. 1A), the steps were as follows:
(1) An antiVEGFscFv plasmid was double-digested with BglII and BsrGI restriction endonucleases. After the digestion was performed at 37°C overnight, an insert fragment was recovered using a gel recovery and purification kit.
(2) An empty vector was double-digested with BglII and BsrGI restriction endonucleases. After the digestion was performed at 37°C overnight, a vector was recovered using a gel recovery and purification kit.
(3) The vector and the insert fragment were ligated at a molar ratio of 1:3 using T4 DNA ligase enzyme, and the ligation product was transformed, plated, and incubated at 37°C overnight.
(4) Single colonies were picked and cultured overnight in a shaker (with kanamycin antibiotic added), plasmids were extracted using a plasmid miniprep kit, and the plasmids were identified by restriction digestion analysis. After successful identification, the plasmids were sent to a company for sequencing. Finally, a successful sequence alignment indicated that the plasmid construction was successful. As shown in FIG. 1B, the map of the constructed plasmid and the result of the restriction digestion analysis are provided.

### Example 2

Transfection of cells with pAAV2-H-antiVEGFscFv (SPH) and pAAV2-antiVEGFscFv (SPL) plasmids: 293T cells were passaged to a 10 cm dish, and transfection was performed after the cells grew stably. The transfection method was as follows: 1.5 mL EP tubes were taken and labeled.

Tube A: 10 µg of an AAV2-antiVEGFscFv plasmid and then 480 µL of Opti-MEM were added, mixed, and left to stand at room temperature for 5 min; Tube B: 20 µg of PEI and then 480 µL of Opti-MEM were added and mixed.

The liquid in Tube A was added to Tube B, mixed, and left to stand at room temperature for 15 min. After 15 min, the liquid was gently mixed and added to a 10 cm dish, which was then placed in a 37°C incubator and cultured for 2-3 days. The cells were harvested for ELISA and Western blot analysis.

### Example 3

In vitro expression of antiVEGF protein by pAAV2-H-antiVEGFscFv and pAAV2-antiVEGFscFv

### (1) Detection of antiVEGF protein expression by Western blot

A 12% SDS-PAGE gel was prepared. Approximately 400 to 500 mL of 1× electrophoresis buffer was added to an electrophoresis tank. Then, samples were loaded into sample wells in sequence. The power was turned on, and electrophoresis was performed at constant voltages (90 V for the stacking gel and 110 V for the resolving gel). The electrophoresis was stopped when the dye front reached the bottom of the gel. A PVDF membrane was activated by immersing it in methanol. The stacking gel was discarded, and a black plate, a fiber pad, a sheet of thick filter paper, the PAGE gel, the PVDF membrane, a sheet of thick filter paper, a fiber pad, and a white plate were sequentially immersed in transfer buffer, and wet transfer was performed at a constant current (200 mA, 120 min). After the membrane transfer was completed, the membrane was blocked with 5% skimmed milk powder. The membrane was washed with 1× TBST on a horizontal shaker for 10 min. The PVDF membrane was completely immersed in a primary antibody solution (the primary antibody was diluted at 1:3000), placed on a horizontal shaker, and incubated at room temperature for 120 min. The membrane was washed 3 times with 1× TBST for 10 min each time to completely remove the residual primary antibody. Then, a diluted secondary antibody (the secondary antibody was diluted at 1:5000) was added, and the membrane was incubated at room temperature for 60 min. The membrane was washed 5 times with 1× TBST for 5 min each time, and then imaged in a pre-cooled chemiluminescence imager.

As shown in FIG. 2A, the Western blot analysis confirmed that an expression of a 26 kDa antiVEGFscFv protein could be detected in both the culture supernatant and the cells transfected with the constructed pAAV2-antiVEGFscFv plasmid.

### (2) Detection of antiVEGF protein expression by ELISA

The expression of the antibody and its binding ability to VEGF were analyzed by ELISA. A 96-well ELISA plate (Fisher, Loughborough, UK) was coated with 1 µg/mL hVEGF and incubated at 4°C overnight. The plate was then blocked by being incubated in 2% BSA at 37°C for 1 h. The culture supernatant was added to the wells in duplicate and incubated at 37°C for 2 h. An anti-anti-VEGF antibody diluted at 1:5000 in PBS blocking buffer was used, and the plate was incubated at room temperature (RT) for 2 h. A secondary antibody diluted at 1:5000 in the blocking buffer was used, and the plate was incubated at room temperature for 1 h. After each of the above steps, the plate was washed 5 times with PBS+0.05% Tween 20. After an HRP substrate was added and color was developed in the dark at 37°C, the reaction was terminated with 2.5 M H₂SO₄, and the absorbance was read at 450 nm on a microplate reader.

As shown in FIG. 2B, the binding of the antiVEGF protein to VEGF in the supernatant of cells transfected with pAAV2-H-antiVEGFscFv and pAAV2-antiVEGFscFv plasmids was detected by the ELISA experiment. The amount of the antiVEGF protein in the supernatant of the pAAV2-antiVEGFscFv group was higher than that in the pAAV2-H-antiVEGFscFv group, thereby leading to a stronger binding signal.

### Example 4

### Virus package and purification

Taking the preparation process of an AAV2-antiVEGFscFv virus as an example:
(1) A total of 700 µg of plasmids was added to a cell factory. The cells were transfected with the plasmids when they reached 80% confluency.

MIX was prepared as follows:
A: In a 50 mL centrifuge tube, a Helper plasmid, a Rep-Cap2 plasmid, and a pAAV2-antiVEGFscFv plasmid were added in equal molar ratios.
B: The concentration ratio of PEI to plasmid was 2:1.

2. A and B were mixed thoroughly by vortex. B was added dropwise to A, mixed by inverting upside down, and left to stand at room temperature for 15 min to obtain the MIX.

3. The cell factory was taken out. Before the MIX was added, the centrifuge tube was inverted upside down twice. The MIX was added dropwise into the cell factory, which was then placed in an incubator for culture.

4. On the second day after the transfection, the supernatant was replaced with an equal amount of serum-free culture medium.

### (2) Harvesting the virus

1. The virus was harvested on the third day after the medium change.
2. The cell factory was shaken vigorously to suspend the cells, which were then transferred to a 50 mL centrifuge tube, centrifuged at 1500 g at 4°C for 10 min, and the supernatant was discarded, leaving the cells at the bottom.
3. An appropriate amount of Lysis buffer was added to the centrifuge tube to resuspend the cells, and the mixture was vortexed.
4. Liquid nitrogen was taken into an appropriate container. The centrifuge tube was flash-frozen in the liquid nitrogen for 10 min, then placed at 37°C until completely thawed, and vortexed for 2 min. Then the centrifuge tube was put into the liquid nitrogen again to repeat this process three times.
5. The centrifuge tube was centrifuged at 1500 g at 4°C for 30 min.

### (3) PEG precipitation

1. The supernatant was transferred to a new 50 mL centrifuge tube, PEG8000 was added, and the mixture was mixed by inverting upside down and precipitated at 4°C overnight.
2. On the next day, the mixture was centrifuged at 1500 g and 4°C for 30 min to pellet the precipitate.
3. The supernatant was discarded.
4. The precipitate was dissolved in 2 mL of PBS.

### (4) Cesium chloride density gradient centrifugation

After the precipitation was completed, the virus was concentrated by cesium chloride density gradient centrifugation. Finally, the titer and purity of the virus were examined by qPCR, silver staining, and genome identification. FIG. 3 shows the results of two rounds of cesium chloride density gradient centrifugation and silver staining of the virus. As shown in FIG. 3A, a clear virus band was obtained after the first round of cesium chloride density gradient centrifugation for 24 h. FIG. 3B shows a clear virus band obtained after the second round of cesium chloride density gradient centrifugation for 20 h; the lower band (b) is the band of viruses with complete genomes, and the upper band of empty capsids (a) did not contain a genome. The virus band was extracted for silver staining to detect virus purity. As shown in FIG. 3C, the three protein bands were all viral capsid proteins. FIG. 3D shows the identification of the viral genome by agarose gel electrophoresis, and the location and size of the bands were correct. The above results indicate that viruses with low impurity and high purity were prepared.

### Example 5

### 1. Infection of cells with AAV2-H-antiVEGFscFv and AAV2-antiVEGFscFv

293T cells were passaged into a 10 cm dish, and viral infection was performed when the cells grew to about 80% confluency.

Good infection efficiency was detected by experiment at a multiplicity of infection (MOI) of 1×10⁵ (denoted as 1e5). An AAV2-antiVEGFscFv/empty vector at an MOI of 1×10⁵ was taken and added dropwise and evenly into culture dishes for infection. The dishes were placed in a 37°C incubator and cultured for 2 to 3 days, and the cells were harvested for western blot analysis. The cells were infected at MOIs of 5e3, 5e4, and 5e5, respectively, and the supernatants were harvested for ELISA.

### 2. In vitro expression of antiVEGF protein by AAV2-antiVEGFscFv

### (1) Detection of antiVEGF protein expression by Western blot

A 12% SDS-PAGE gel was prepared. Approximately 400 to 500 mL of 1× electrophoresis buffer was added to an electrophoresis tank. Then, samples were loaded into sample wells in sequence. The power was turned on, and electrophoresis was performed at constant voltages (90 V for the stacking gel and 110 V for the resolving gel). The electrophoresis was stopped when the dye front reached the bottom of the gel. A PVDF membrane was activated by immersing it in methanol. The stacking gel was discarded, and a black plate, a fiber pad, a sheet of thick filter paper, the PAGE gel, the PVDF membrane, a sheet of thick filter paper, a fiber pad, and a white plate were sequentially immersed in transfer buffer, and wet transfer was performed at a constant current (200 mA, 120 min). After the membrane transfer was completed, the membrane was blocked with 5% skimmed milk powder. The membrane was washed with 1× TBST on a horizontal shaker for 10 min. The PVDF membrane was completely immersed in a primary antibody solution (the primary antibody was diluted at 1:3000), placed on a horizontal shaker, and incubated at room temperature for 120 min. The membrane was washed 3 times with 1× TBST for 10 min each time to completely remove the residual primary antibody. Then, a diluted secondary antibody (the secondary antibody was diluted at 1:5000) was added, and the membrane was incubated at room temperature for 60 min. The membrane was washed 5 times with 1× TBST for 5 min each time, and then imaged in a pre-cooled chemiluminescence imager.

As shown in FIG. 4B, Western blot analysis confirmed that the expression of antiVEGFscFv could be detected in both the culture supernatant and the cells infected with the AAV2-antiVEGFscFv virus.

### (2) Detection of antiVEGF protein expression by ELISA

The anti-VEGF binding ability of the antibody was analyzed by ELISA. A 96-well ELISA plate (Fisher, Loughborough, UK) was coated by being incubated at 4°C overnight with 1 µg/mL hVEGF (Abcam, Cambridge, UK) in PBS. The plate was then blocked by being incubated in 2% BSA at 37°C for 1 h. Samples for the anti-VEGF test were prepared in a blocking buffer, added to the wells in duplicate, and incubated at 37°C for 3 h. An anti-antiVEGFscFv antibody diluted at 1:2000 in PBS blocking buffer, was used, and the plate was incubated at room temperature (RT) for 2 h. An anti-Rabbit antibody diluted at 1:5000 in the blocking buffer, was used, and the plate was incubated at room temperature for 1 h. After each of the above steps, the plate was washed 5 times with PBS+0.05% Tween 20. After an HRP substrate was added and color was developed in the dark at 37°C, the reaction was terminated with 2.5 M H₂SO₄, and the absorbance was read at 490 nm on a microplate reader.

As shown in FIG. 4A, ELISA confirmed that the expression of antiVEGFscFv could be detected in the culture supernatant of cells infected with the AAV2-antiVEGFscFv virus, and the antibody expression level increased with the increase of MOI.

### Example 6

### Inhibitory effect of AAV2-antiVEGFscFv virus on the proliferation of HUVEC cells

HUVECs in the logarithmic growth phase were taken, digested with 0.25% trypsin, and resuspended in FBS-free ECM culture medium. The cell suspension density was adjusted to 1×10⁵ cells/mL, and the cell suspension was seeded into a 96-well plate at 100 µL per well. The cells were cultured and starved for 24 h using FBS-free ECM culture medium in an incubator with 5% CO₂ at 37°C.

### empty vector

The original cell culture medium was removed. 100 µL of FBS-free ECM culture medium was added as a blank control group (denoted as Ctrl);
on the basis of the blank control group, 100 µL of VEGF165 (at a concentration of 50 ng/mL) was added in addition as a positive control group (denoted as VEGF 50 ng/mL);
on the basis of the positive control group, Beo (brolucizumab, Atagenix Cat# ATAD00374) was added as drug intervention groups at concentrations of 2 µg/mL, 200 ng/mL, and 20 ng/mL, respectively, with 3 parallel wells per group (denoted as VEGF+Beo 2 µg/mL, VEGF+Beo 200 ng/mL, and VEGF+Beo 20 ng/mL, respectively);
an empty vector was diluted with FBS-free ECM culture medium and added to cells treated with VEGF165, at gradient concentrations of 5×10⁵, 5×10⁴, and 5×10³, respectively (denoted as VEGF+empty vector MOI:5e5, VEGF+empty vector MOI:5e4, and VEGF+empty vector MOI:5e3, respectively); and
AAV2-antiVEGFscFv was diluted with FBS-free ECM culture medium and added to cells treated with VEGF165, at gradient concentrations of 5×10⁵, 5×10⁴, and 5×10³, respectively (denoted as VEGF+AAV2-antiVEGFscFv MOI:5e5, VEGF+AAV2-antiVEGFscFv MOI:5e4, and VEGF+AAV2-antiVEGFscFv MOI:5e3, respectively).

All groups were further cultured for 48 h in an incubator with 5% CO₂ at 37°C. 10 µL of CCK8 was added to each well, and the plate was further cultured for 2 h in the incubator with 5% CO₂ at 37°C. The OD value of each well was measured at a wavelength of 450 nm with a microplate reader. The experiment was repeated 3 times.

HUVECs are human umbilical vein endothelial cells, and neovascularization is primarily accompanied by the proliferation of endothelial cells. To explore whether the virus has an inhibitory effect on the proliferation of HUVEC cells, HUVEC cells were evenly seeded in a 96-well plate, and cell proliferation was then intervened with different viruses and drugs. As shown in FIG. 5, it can be seen that compared to the negative control group (the empty vector group), cell proliferation in the experimental groups to which the AAV2-antiVEGFscFv virus was added was significantly reduced. It indicates that the virus has a certain inhibitory effect on the proliferation function of HUVEC cells.

### Example 7

Inhibitory effect of AAV2-antiVEGFscFv virus on the tube formation ability of HUVEC cells

Matrigel (growth factor reduced type) was thawed on ice to prevent premature polymerization. A 96-well cell culture plate was placed on ice, and 50 µL of matrigel was added to each well, avoiding air bubbles and ensuring that the matrigel covered the entire bottom of the well. The matrigel was allowed to polymerize at 37°C for 60 min. A suspension of HUVECs in the logarithmic growth phase was prepared, and the suspension concentration was adjusted to 7×10³ cells/mL. The cells were mixed with different drug incubation solutions (grouping was the same as in Example 6), and were immediately seeded into the matrigel-coated plate, which was then placed in an incubator with 5% CO₂ at 37°C for culture. The cells were observed under a microscope at 0, 6, and 9 h, samples were taken and photographed, and the experiment was repeated 3 times.

To explore whether the virus had an inhibitory effect on the tube formation function of HUVEC cells, matrigel was added to a 96-well plate, HUVEC cells were evenly seeded, and cell proliferation was then intervened with different viruses and drugs. As shown in FIG. 6, it can be seen that compared to the negative control group, the tube formation of the experimental groups to which the AAV2-antiVEGFscFv virus was added was significantly reduced, indicating an effect superior to that of the negative control group. It indicates that the virus had a certain inhibitory effect on the tube formation function of HUVEC cells.

### Example 8

### Expression of AAV2-antiVEGFscFv virus in mouse eyes

To detect whether AAV2-antiVEGFscFv could successfully express an antiVEGF protein in the eyeballs of C57BL/6 mice, each eye of a wild-type C57BL/6 mouse received a single intravitreal injection of 5×10⁸ GC of AAV2-antiVEGFscFv or an empty vector. 14 days after the intravitreal injection of the vectors, the genomic DNA and RNA of the mouse eyeballs were extracted and quantified by real-time fluorescent quantitative PCR using absolute quantification and relative quantification methods, respectively.

Total RNA was extracted from the eyeballs using a TRIzol reagent. The RNA concentration was then measured using Nano Photometer N50 Touch. Different RNAs were diluted to 200 ng/µL and then reverse-transcribed into cDNA using PrimeScript RT Master Mix. Then, fluorescent quantification was performed. The primer sequences were as follows:
scfv-VH-F: 5'-GCTTCAGCCTGACCGACTAC-3' (forward)
scfv-VH-R: 5'-CTGATGGTGAACCTGCCCTT-3' (reverse)
GAPDH: 5'-TGACTTCAACAGCGACACCCA-3' (forward)
GAPDH: 5'-CACCCTGTTGCTGTAGCCAAA-3' (reverse)

Genomic DNA (gDNA) was extracted using a DNeasy Blood & Tissue Kit and quantified by qPCR. Relative expression values were calculated using the relative quantification method (2^{-ΔΔCt})

The results are shown in FIG. 7. FIG. 7A shows the absolute quantification by qPCR of genome extracted from mouse eyeballs two weeks after the intravitreal injection of the empty vector or AAV2-antiVEGFscFv. It can be seen that for the antiVEGFscFv genome, the level in the experimental group was much higher than that in the control group, with statistical significance. FIG. 7B shows the relative quantification by real-time PCR of RNA extracted from the eyeballs of wild-type mice and mice two weeks after the intravitreal injection of the virus. Compared to the control group of wild-type mice, the expression in the experimental group injected with the virus was about 100 times higher, with statistical significance.

### Example 9

Inhibitory effect of AAV2-antiVEGFscFv virus on ocular neovascularization in CNV mice

To explore whether the virus has an inhibitory effect on ocular neovascularization in a laser-induced choroidal neovascularization (CNV) model in mice, 4-week-old wild-type C57BL/6 mice were used. The mice were given a unilateral intravitreal injection of 2 µL (5×10⁸) of an AAV2-antiVEGFscFv virus or an empty vector, and the contralateral eye was injected with an equal volume of PBS. Two weeks later, lesions were caused by laser-induced ocular neovascularization in the mice. One week later, fundus imaging and fluorescein angiography were performed.

As shown in FIG. 8, the area of the neovascular lesion induced by laser in the eyes of mice injected with the AAV2-antiVEGFscFv virus was significantly smaller than that of the lesion in the control group mice. It indicates that the AAV2-antiVEGFscFv virus had a certain inhibitory effect on ocular neovascularization in CNV mice.

### Example 10

### Inhibitory effect of AAV2-antiVEGFscFv virus on ocular neovascularization in CNV rats

To explore whether the virus has an inhibitory effect on ocular neovascularization in a laser-induced choroidal neovascularization (CNV) model in rats, 12-week-old wild-type BN rats were used. The rats were given a unilateral intravitreal injection of 4 µL (1×10⁹) of an AAV2-antiVEGFscFv virus or an empty vector, and the contralateral eye was injected with an equal volume of PBS. Two weeks later, lesions were caused by laser-induced ocular neovascularization in the rats. Fluorescein angiography was performed on D2, D7, D14, D21, and D28 after the laser induction, respectively.

As shown in FIG. 9, the area of the neovascular lesion induced by laser in the eyes of rats injected with the AAV2-antiVEGFscFv virus was significantly smaller than that of the lesion in the control group rats. It indicates that the AAV2-antiVEGFscFv virus had a certain inhibitory effect on ocular neovascularization in the age-related macular degeneration rat model.

### Example 11

### Inhibitory effect of AAV2-antiVEGFscFv virus on ocular neovascularization in RhortTA/Tet-VEGFA double transgenic mice

To detect the inhibitory effect of AAV2-antiVEGFscFv on neovascularization in a Rho-rtTA/Tet-VEGFA double transgenic mouse model, an intravitreal injection of 2 µL (5×10⁸) of an AAV2-antiVEGFscFv virus was given. Mice injected with an equal volume of an empty vector (5×10⁸) virus served as a control group. Eyeballs were collected two weeks later.

As shown in FIG. 10, FIG. 10A shows inhibition of neovascularization verified by retinal flat-mount and lectin staining, FIG. 10B shows inhibition of retinal detachment verified by eyeball tissue sections and immunohistofluorescence, and FIG. 10C shows inhibition of vascular leakage by the virus as demonstrated by fluorescein angiography.

### Example 12

### Inhibitory effect of AAV2-antiVEGFscFv virus on ocular neovascularization in OIR mice

To detect the inhibitory effect of AAV2-antiVEGFscFv on neovascularization in an oxygen-induced retinopathy (OIR) mouse model, eyeballs were taken out 5 days after the intravitreal injection of 1 µL (2.5×10⁸) of an AAV2-antiVEGFscFv virus or an empty vector virus, respectively. FIG. 11 shows the inhibitory effect on neovascularization as demonstrated by retinal flat-mount and lectin staining. As shown in the figure, compared to normoxia mice, the retinas of the OIR mice showed significant central vascular occlusion, non-perfusion areas, and vascular proliferation and leakage. In OIR mice injected with AAV2-antiVEGFscFv, the above lesions were significantly reduced, while there was no improvement in OIR mice injected with the empty vector. It indicates that the AAV2-antiVEGFscFv virus had a certain inhibitory effect on ocular neovascularization in the oxygen-induced retinopathy mouse model.

### Example 13

### Inhibitory effect of AAV2-antiVEGFscFv virus on lesions in a VEGF-induced retinal vascular leakage rabbit model

To detect the inhibitory effect of AAV2-antiVEGFscFv on retinal neovascularization and leakage, on D0, a subretinal injection of 50 µL (1.5×10¹⁰) of an AAV2-antiVEGFscFv virus, an empty vector virus, or a positive control (an anti-neovascularization antibody drug) was performed. On D14, VEGF was injected subretinally to induce retinal vascular leakage. On D21, fluorescein angiography was performed to demonstrate the inhibition of neovascularization and leakage by the virus. As shown in FIG. 12, in rabbits injected with AAV2-antiVEGFscFv, angiographic leakage was significantly reduced, while there was no improvement in rabbits injected with the empty vector. It indicates that the AAV2-antiVEGFscFv virus had a certain inhibitory effect on lesions in the VEGF-induced retinal vascular leakage rabbit model.

The above are only preferred embodiments of the present disclosure. It should be noted that for a person of ordinary skill in the art, several improvements and modifications can also be made without departing from the principles of the present disclosure. These improvements and modifications should also be considered within the protection scope of the present disclosure.

## Claims

1. An anti-vascular endothelial growth factor (VEGF) single-chain antibody, comprising:
three light chain complementarity-determining regions (CDRs) having amino acid sequences as set forth in SEQ ID NOs: 1, 2 and3, respectively; and
three heavy chain CDRs having amino acid sequences as set forth in SEQ ID NOs: 4, 5 and 6, respectively.

2. The anti-VEGF single-chain antibody according to claim 1, wherein the anti-VEGF single-chain antibody comprises a light chain variable region, a linker, and a heavy chain variable region; wherein:
the heavy chain variable region has an amino acid sequence as set forth in SEQ ID NO: 7; and
the light chain variable region has an amino acid sequence as set forth in SEQ ID NO: 8.

3. The anti-VEGF single-chain antibody according to claim 1 or 2, wherein the anti-VEGF single-chain antibody comprises, from N-terminal to C-terminal, a secretory signal peptide, a light chain variable region, a linker, and a heavy chain variable region, wherein:
the secretory signal peptide has an amino acid sequence as set forth in SEQ ID NO: 9; and
the linker is (G₄S)₄.

4. A nucleic acid encoding the anti-VEGF single-chain antibody according to any one of claims 1 to 3.

5. The nucleic acid according to claim 4, wherein the nucleic acid comprises:
a nucleotide sequence encoding a light chain variable region as set forth in SEQ ID NO: 10; and
a nucleotide sequence encoding a heavy chain variable region as set forth in SEQ ID NO: 11.

6. The nucleic acid according to claim 4, wherein the nucleic acid encoding the anti-VEGF single-chain antibody has a nucleotide sequence as set forth in SEQ ID: NO: 12.

7. An expression unit comprising a promoter and the nucleic acid according to any one of claims 4 to 6.

8. The expression unit according to claim 7, wherein the promoter is a chicken β-actin promoter.

9. A recombinant vector comprising the nucleic acid according to any one of claims 4 to 6 or the expression unit according to claim 7 or 8.

10. The recombinant vector according to claim 9, wherein the recombinant vector successively comprises a 5' terminal inverted repeat sequence, a promoter sequence, the nucleic acid according to any one of claims 4 to 6, a T2A sequence, a BGHpolyA signal sequence, and a 3' terminal inverted repeat sequence.

11. The recombinant vector according to claim 9, wherein the recombinant vector has a backbone vector of pAAV2.

12. A host, comprising a nucleic acid according to any one of claims 4 to 6 integrated into a genome thereof; or transfected or transformed with the recombinant vector according to any one of claims 9 to 11.

13. A recombinant adeno-associated virus obtained by culturing the host according to claim 12.

14. Use of any one of I) to VI), in the manufacture of a medicament for ameliorating an ocular neovascularization-related disease:
I) the anti-VEGF single-chain antibody according to any one of claims 1 to 3;
II) the nucleic acid according to any one of claims 4 to 6;
III) the expression unit according to claim 7 or 8;
IV) the recombinant vector according to any one of claims 9 to 11;
V) the host according to claim 12;
VI) the recombinant adeno-associated virus according to claim 13.

15. The use according to claim 14, wherein the ocular neovascularization-related disease includes neovascular age-related macular degeneration, diabetic retinopathy, retinopathy of prematurity, corneal neovascularization, retinal vascular occlusion, and/or neovascular glaucoma.

16. A medicament for ameliorating an ocular neovascularization-related disease, comprising the recombinant adeno-associated virus according to claim 13.

17. The medicament according to claim 16, wherein the medicament is in a dosage form of an injection, and the injection is an intravitreal injection, a subretinal injection, or a suprachoroidal injection.
